# EUROPEAN PATENT APPLICATION

(11) **EP 4 502 174 A1**
(43) Date of publication of application: **05.02.2025**
(21) Application number: 23780722.7
(22) Date of filing: 29.03.2023
(51) Int. Cl.: C12Q 1/34

(54) **METHOD FOR MEASURING LYSOPHOSPHOLIPASE D ACTIVITY, SENSITIVITY ENHANCEMENT AGENT FOR LYSOPHOSPHOLIPASE D ACTIVITY MEASUREMENT, COMPOSITION, AND KIT**

(30) Priority: 31.03.2022 JP 2022058195
(71) Applicant: FUJIFILM Corporation, Tokyo 106-8620 (JP)
(72) Inventor: OKUBO, Kimaru, Amagasaki-shi, Hyogo 661-0963 (JP); ITAI, Tomokazu, Amagasaki-shi, Hyogo 661-0963 (JP)
(74) Representative: Dehns Germany Partnerschaft mbB
(86) International application number: PCT/JP2023/012871
(87) International publication number: WO 2023/190714

(57) **Abstract**

There is provided an activity measurement method of lysophospholipase D, including measuring a lysophospholipase D activity in a presence of ferrocyanide, a sensitivity improver for activity measurement of lysophospholipase D activity, which contains ferrocyanide, a composition for an activity measurement method of lysophospholipase D, and a kit for activity measurement of lysophospholipase D.

## Description

### Technical Field

The present invention relates to an activity measurement method of lysophospholipase D, a sensitivity improver for activity measurement of lysophospholipase D, a composition, and a kit.

### Background Art

The lysophospholipase D is an enzyme that plays an important role as a lysophosphatidic acid (hereinafter, may be abbreviated as LPA) producing enzyme, in a living body.

It has been reported that the lysophospholipase D activity in the blood increases with liver fibrosis and LPA has attracted attention as a liver fibrosis marker. In addition, LPA is a physiologically active lipid that affects the movement, proliferation, and survival in various cell types, and is considered to play a role in promoting various diseases such as inflammation such as arthritis in a living body, neurodegeneration, neuropathic pain, thyroid disease, and pathological states including cancer. Therefore, the causal relationship between lysophospholipase D, which is a production enzyme of LPA, and various diseases has been studied.

For example, Non-Patent Document 1 discloses that autotaxin, which is one type of lysophospholipase D, dramatically increases chemotaxis and proliferation of a plurality of different cell strains in the presence of lysophosphatidylcholine (LPC), and that several cancer cell strains massively release LPC, which is a substrate of ATX, into a culture solution.

### Prior Art Documents

### Non-Patent Documents

Non-Patent Document 1: J Cell Biol. 2002 Jul 22;158(2):227-33

### SUMMARY OF THE INVENTION

### Object to be solved by the invention

As described above, since the causal relationship with various diseases is suggested for the lysophospholipase D, it is considered that, for example, measurement of the activity of lysophospholipase D in a specimen such as blood is useful for the discovery of various diseases, the prediction of the degree of the disease, and the like.

However, the conventional lysophospholipase D activity measurement method may take time for the measurement, and thus the measurement of the activity of lysophospholipase D has not been clinically applied. It is considered that this is because the sensitivity of the lysophospholipase D activity measurement in the conventional method is not sufficient.

An object of the present invention is to provide an activity measurement method of a lysophospholipase D, which has excellent sensitivity in the activity measurement, a sensitivity improver for lysophospholipase D activity measurement, a composition for activity measurement of a lysophospholipase D, which has excellent sensitivity in the activity measurement, and a kit for activity measurement of a lysophospholipase D, which has excellent sensitivity in the activity measurement.

### Means for solving the object

The present inventors have conducted studies and found that the sensitivity of the lysophospholipase D activity measurement is improved by coexisting a ferrocyanide during the measurement of the lysophospholipase D activity, and have completed the present invention.

Representative aspects of the present invention are shown below. However, the present invention is not limited thereto.
<1> An activity measurement method of lysophospholipase D, comprising measuring lysophospholipase D activity possessed by the lysophospholipase D, in a presence of a ferrocyanide.
<2> The activity measurement method of lysophospholipase D according to <1>, in which the ferrocyanide is potassium ferrocyanide or sodium ferrocyanide.
<3> The activity measurement method of lysophospholipase D according to <1> or <2>, in which the measurement includes bringing the lysophospholipase D into contact with a substrate to produce a product.
<4> The activity measurement method of lysophospholipase D according to <3>, in which the substrate is lysophosphatidylcholine and the product is choline.
<5> The activity measurement method of lysophospholipase D according to any one of <1> to <4>, in which the lysophospholipase D is autotaxin.
<6> The activity measurement method of lysophospholipase D according to any one of <1> to <5>, in which a concentration of the ferrocyanide in the measurement of the lysophospholipase D activity is 0.005 mmol/l or more.
<7> A sensitivity improver for activity measurement of lysophospholipase D, comprising a ferrocyanide.
<8> A composition for activity measurement of lysophospholipase D, comprising a ferrocyanide and a substrate of lysophospholipase D.
<9> The composition according to <8>, in which the substrate is lysophosphatidylcholine.
<10> The composition according to <8> or <9>, in which a concentration of the ferrocyanide in the activity measurement is 0.005 mmol/l or more.
<11> A kit for activity measurement of lysophospholipase D, comprising the composition according to any one of <8> to <10> or two or more reagents in which components contained in the composition are distributed.

### Effect of the invention

According to the present invention, there are provided an activity measurement method of lysophospholipase D, which has excellent sensitivity in the activity measurement, a sensitivity improver for lysophospholipase D activity measurement, a composition for activity measurement of lysophospholipase D, which has excellent sensitivity in the activity measurement, and a kit for activity measurement of lysophospholipase D, which has excellent sensitivity in the activity measurement.

### Embodiments for carrying out the invention

### (Activity measurement method of lysophospholipase D)

The activity measurement method of lysophospholipase D according to the embodiment of the present invention includes measuring the lysophospholipase D activity of the lysophospholipase D in the presence of ferrocyanide.

According to the present invention, there is provided an activity measurement method of lysophospholipase D having excellent sensitivity in an activity measurement.

Here, for example, J Cell Biol. 2002 Jul 22;158(2):227-33 discloses a measuring method, as a method for measuring lysophospholipase D activity of autotaxin (ATX), detecting choline generated by bringing lysophosphatidylcholine (LPC) as a substrate into contact with autotaxin in a sample.

Specifically, in J Cell Biol. 2002 Jul 22;158(2):227-33, it is described that 1 to 50 µl of a specimen is incubated at 37°C for 1 hour together with 1 mM LPC (egg-derived) in the presence of 100 mM Tris-HCl, pH 9.0, 500 mM NaCl, 5 mM MgCl₂, and 0.05% Triton X-100, and the liberated choline is detected by an enzymatic spectrophotometry using choline oxidase (Asahi Kasei), horseradish peroxidase (Toyobo), and TOOS reagent (N-ethyl-N-(2-hydroxy-3-sulphoprolyl)-3-methylaniline) as a hydrogen donor.

However, it was found that the method described in J Cell Biol. 2002 Jul 22;158(2):227-33 has low sensitivity and takes time for measurement in a case of actually measuring low concentrations of lysophospholipase D such as autotaxin in a specimen.

Therefore, the present inventors have conducted studies and found that the measurement sensitivity of the lysophospholipase D activity is improved in a case where a ferrocyanide is made to coexist in the activity measurement of the lysophospholipase D.

In addition, the present inventors have found that, simultaneous reproducibility is excellent in a case where ferrocyanide is made to coexist in the activity measurement of lysophospholipase D.

The excellent simultaneous reproducibility means that the variation in the measurement results is small in a case where the same specimen is repeatedly measured in a short time.

Hereinafter, the activity measurement method of lysophospholipase D according to the embodiment of the present invention will be described in detail. In the present invention, mmol/l is also described as mM.

### <Measurement of lysophospholipase D activity>

The activity measurement method of lysophospholipase D according to the embodiment of the present invention includes measuring the lysophospholipase D activity of the lysophospholipase D in the presence of ferrocyanide.

### [Ferrocyanide]

The ferrocyanide is not particularly limited, and a known ferrocyanide can be used, but potassium ferrocyanide or sodium ferrocyanide is preferable.

In addition, the ferrocyanide may be ionized into a ferrocyanide ion ([Fe(CN)₆]⁴⁻) and a cation (for example, a metal cation such as K⁺ and Na⁺) during the measurement of the lysophospholipase D activity.

The concentration of the ferrocyanide in a case of measuring the lysophospholipase D activity is not particularly limited, but is preferably 0.005 mM or more, more preferably 0.050 mM or more, and still more preferably 0.500 mM or more. The upper limit of the above-described concentration is not particularly limited, but is, for example, preferably 30 mM or less and more preferably 20 mM or less.

The ferrocyanide may be used alone as one type or in combination of two or more types thereof. In a case where two or more types thereof are used in combination, it is preferable that the total concentration thereof is within the above-described range.

### [Measuring method]

The measuring method of the lysophospholipase D activity is not particularly limited as long as that it is performed in the presence of the ferrocyanide, and a known method can be used without limitation.

The lysophospholipase D to be measured is not particularly limited, but is preferably autotaxin (ATX) and more preferably human autotaxin.

Human autotaxin is a glycoprotein that has been isolated as a factor having cell motility promoting activity from a culture supernatant of human malignant melanoma cell strain A2058 by M. L. Stracke et al. in 1992 (J. Biol. Chem. 1992, 267:2524-2529).

Here, the measurement of the lysophospholipase D activity is preferably a measuring method of the lysophospholipase D activity in a specimen or a standard product.

The specimen is not particularly limited as long as it contains or may contain lysophospholipase D. In addition, the specimen may not contain the lysophospholipase D as a result.

Specific examples of the specimen include body fluids (for example, blood, urine, cerebrospinal fluid, pleural effusion, ascites, puncture fluid, an extracted product thereof, or the like), tissues (for example, biopsy tissue, an extracted product thereof, or the like), a lysophospholipase D solution, cultured cells or an extracted product thereof, and a cell culture supernatant, and blood is preferable.

In addition, the specimen may be subjected to a treatment such as separation or purification in advance. For example, the blood specimen may be used by using separated serum or plasma. In addition, in a case where a compound corresponding to the lysophospholipase D and a product generated from the substrate, which is described later, is also included in the specimen, a treatment such as removing this compound from the specimen in advance may be performed.

The measurement of the lysophospholipase D activity preferably includes bringing the above-described lysophospholipase D into contact with a substrate to produce a product. In such an aspect, for example, the lysophospholipase D activity can be measured by measuring the production amount of the product per unit time.

The above-described contact between the lysophospholipase D and the substrate is preferably performed in a liquid, and more preferably performed in a pH buffer solution. Examples of the pH buffer solution include a buffer solution containing a phosphate buffer agent, a citrate buffer agent, an acetate buffer agent, a tris-hydrochloride buffer agent, a good buffer agent such as TAPS, Bicine, PIPES, MES, and HEPES, or another compound used as a chemical buffer agent, and the like.

A pH of the liquid during the above-described contact is not particularly limited, and examples thereof include a pH of 6.0 to 11.0.

The substrate is not particularly limited as long as it is a substrate of the lysophospholipase D, and a lysophosphatidic acid ester which is a substrate of the lysophospholipase D is preferable, lysophosphatidylcholine or lysophosphatidylglycerol is more preferable, and lysophosphatidylcholine is still more preferable.

In addition, as the substrate of the lysophospholipase D, for example, a substrate labeled for imparting functions such as fluorescence and luminescence, such as a compound which is cleaved by the action of lysophospholipase D and emits fluorescence after the cleavage, may be used.

As such a labeled substrate, a known substrate in the related art can be used without particular limitation, and for example, FS-3 (manufactured by Echelon Biosciences Inc.) or the like can be used.

In the present invention, it is preferable that the above-described substrate is lysophosphatidylcholine and the above-described product is choline.

Examples of the above-described lysophosphatidylcholine include a compound represented by Formula (LPC-1).

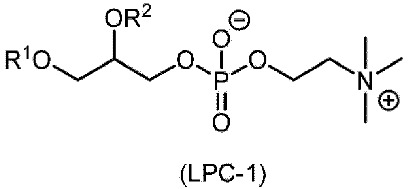

One of R¹ and R² is a hydrogen atom and the other is an alkylcarbonyl group. The number of carbon atoms in the above-described alkylcarbonyl group is preferably 2 to 30 and more preferably 10 to 20.

Examples of the compound represented by Formula (LPC-1) include a compound having the following structure, but the compound is not limited thereto.

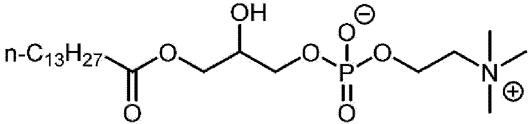

The concentration of the substrate during the above-described contact is not particularly limited, and may be determined in consideration of the solubility of the substrate itself, the measurement sensitivity, the simultaneous reproducibility of the measurement, and the like, but is, for example, preferably 0.1 to 20 mM and more preferably 0.5 to 10 mM.

The substrate may be used alone as one type or in combination of two or more types thereof. In a case where two or more types thereof are used in combination, it is preferable that the total concentration thereof is within the above-described range.

In a case where the above-described contact is performed in a liquid, the above-described contact may be performed in the presence of a surfactant for the purpose of assisting in the dissolution of the substrate, dispersing the color developing reagent to be generated, and the like. The surfactant is not particularly limited, and may be an ionic surfactant or a nonionic surfactant, and examples thereof include a nonionic surfactant such as Triton (registered trademark) X-100.

The concentration of the surfactant is not particularly limited, but is, for example, preferably 0.001% to 1.0% (w/v) with respect to the volume of the reaction solution. In the present specification, % (w/v) indicates a proportion of the content (g) of a compound with respect to 100 mL of the volume of the liquid.

The surfactant may be used alone as one type or in combination of two or more types thereof. In a case where two or more types thereof are used in combination, it is preferable that the total concentration thereof is within the above-described range.

The temperature at the time of the above-described contact is not particularly limited, and may be a temperature in the vicinity of an optimum temperature of the lysophospholipase D to be measured, and examples thereof include 20°C to 45°C.

In the measurement of the lysophospholipase D activity, the production amount of the above-described product is measured, for example, by an oxidase-peroxidase color developing system. The measurement by the oxidase-peroxidase color developing system is a method of performing an enzyme reaction on the above-described product to generate hydrogen peroxide, and reacting the hydrogen peroxide with a color developing reagent in the presence of peroxidase to perform colorimetric quantification.

Since the degree of color development (for example, measured as an absorbance) depends on the concentration of the product, the concentration of the product and the amount of the product can be quantified from the degree of color development.

In a case where there is no appropriate enzyme that catalyzes a reaction of directly oxidizing the product to generate hydrogen peroxide, it is also possible to measure the amount of the measurement target substance by appropriately designing a conjugate reaction in which an enzyme that catalyzes a reaction of converting the product into a substrate capable of generating hydrogen peroxide (the product may be converted into the substrate by a plurality of stages) and an oxidase are combined.

For example, in a case where the substrate is lysophosphatidylglycerol and the product is glycerol, glycerol may be converted into glycerol-3-phosphate by glycerol kinase and adenosine triphosphate (ATP), and then hydrogen peroxide may be generated by glycerol-3-phosphate oxidase which is an oxidase.

That is, an aspect in which an oxidase of the product, a color developing reagent, and a peroxidase are included during the above-described contact in the activity measurement method of lysophospholipase D according to the embodiment of the present invention, is preferable.

As the color developing reagent, a combination of a hydrogen donor such as a trinder reagent and a coupler thereof, a combination of a hydrogen acceptor such as a nitrotetrazolium blue and a coupler thereof, a compound that emits by itself such as a leuco coloring agent such as sodium 10-(carboxymethylaminocarbonyl)-3,7-bis(dimethylamino)phenothiazine, or the like, can be used.

For example, an aspect in which an oxidase of the product, a trinder reagent, a trinder reagent coupler, and a peroxidase are included during the above-described contact in the activity measurement method of lysophospholipase D according to the embodiment of the present invention, is preferable.

The above-described oxidase of the product is not particularly limited, and may be selected depending on the type of the product.

Examples thereof include choline oxidase in a case where the product is choline.

The above-described choline oxidase is not particularly limited as long as it produces hydrogen peroxide from choline. Examples thereof include choline oxidase derived from a microorganism' or an Arthrobacter species.

The above-described choline oxidase may be a natural product, a chemically or biochemically synthesized product, or a product produced by genetic engineering. In addition, a commercially available product can be used.

Examples thereof include glycerol-3-phosphate oxidase in a case where the product is glycerol. However, in a case of using glycerol-3-phosphate oxidase, as described above, an enzyme and a substrate (for example, glycerol kinase and ATP) for further producing glycerol-3-phosphate from glycerol are used.

The concentration of the above-described oxidase of the product during the above-described contact may be a concentration sufficient to react with the generated product, and may be determined in consideration of the measurement sensitivity, the simultaneous reproducibility of the measurement, and the like, but is preferably 0.01 to 200 U/mL and more preferably 0.1 to 100 U/mL.

The oxidase of the product may be used alone as one type or in combination of two or more types thereof. In a case where two or more types thereof are used in combination, it is preferable that the total concentration thereof is within the above-described range. In addition, the oxidase of the product may be immobilized on a container (for example, a well of a plate), beads (for example, resin beads), or the like, which is used for the measurement.

The above-described trinder reagent is not particularly limited, and examples thereof include phenol and a derivative thereof, an aniline derivative, naphthol and a derivative thereof, naphthylamine and a derivative thereof, and the like.

Specific examples of the trinder reagent include N-ethyl-N-sulfopropyl-3-methoxyaniline (ADPS), N-ethyl-N-sulfopropylaniline (ALPS), N-ethyl-N-sulfopropyl-3-methylaniline (TOPS), N-ethyl-N-(2-hydroxy-3-sulfopropyl)-3-methoxyaniline (ADOS), N-ethyl-N-(2-hydroxy-3-sulfopropyl)-3,5-dimethoxyaniline (DAOS), N-(2-hydroxy-3-sulfopropyl)-3,5-dimethoxyaniline (HDAOS), N-ethyl-N-(2-hydroxy-3-sulfopropyl)-3,5-dimethylaniline (MAOS), N-ethyl-N-(2-hydroxy-3-sulfopropyl)-3-methoxyaniline (TOOS), N-(3-sulfopropyl)-3-methoxy-5-methylaniline (HMMPS), N,N-bis(4-sulfobutyl)-3-methylaniline (TODB), and the like.

The concentration of the above-described trinder reagent during the above-described contact is not particularly limited, and may be determined in consideration of the measurement sensitivity, the simultaneous reproducibility of the measurement, and the like, but is preferably 0.1 to 20 mM and more preferably 0.5 to 10 mM.

The trinder reagent may be used alone as one type or in combination of two or more types thereof. In a case where two or more types thereof are used in combination, it is preferable that the total concentration thereof is within the above-described range.

Examples of the coupler of the above-described trinder reagent include 4-aminantipyrine (4-AA) or a derivative thereof, vanillin diamine sulfonate, methylbenzothiazolinone hydrazone (MBTH), methylbenzothiazolinone hydrazone sulfonate (SMBTH), and the like, and from the viewpoint of the stability of the coupler itself, 4-aminantipyrine is preferable.

The concentration of the above-described coupler of the trinder reagent during the above-described contact is not particularly limited, and may be determined in consideration of the measurement sensitivity, the simultaneous reproducibility of the measurement, and the like, but is preferably 0.1 to 20 mM and more preferably 0.5 to 10 mM.

The coupler of the trinder reagent may be used alone as one type or in combination of two or more types thereof. In a case where two or more types thereof are used in combination, it is preferable that the total concentration thereof is within the above-described range.

The peroxidase is not particularly limited as long as it is an enzyme which oxidatively condenses a trinder reagent and a coupler of the trinder reagent. Examples thereof include a peroxidase derived from horseradish or derived from a microorganism such as bacteria, mold, or the like. The peroxidase may be a natural product, a chemically or biochemically synthesized product, or a product produced by genetic engineering. In addition, a commercially available product can be used.

The concentration of the peroxidase may be a concentration sufficient to react with the generated hydrogen peroxide, and may be determined in consideration of the measurement sensitivity, the simultaneous reproducibility of the measurement, and the like, but is preferably 0.01 to 200 U/mL and more preferably 0.1 to 100 U/mL.

The peroxidase may be used alone as one type or in combination of two or more types thereof. In a case where two or more types thereof are used in combination, it is preferable that the total concentration thereof is within the above-described range. In addition, the peroxidase may be immobilized on a container (for example, a well of a plate), beads (for example, resin beads), or the like, which is used for the measurement.

In addition, the production amount of the product may be measured by another known method. Specifically, the product may be amplified by an enzyme cycling method in which the product is included in a reaction mechanism, and the production amount thereof may be measured.

Hereinafter, representative preferred aspects of the activity measurement method of the lysophospholipase D according to the embodiment of the present invention will be described, but the present invention is not limited thereto.

### [First aspect of measurement of lysophospholipase D activity]

The measurement of the lysophospholipase D activity of the present invention is preferably performed by adding ferrocyanide, a trinder reagent, a choline oxidase, a peroxidase, lysophosphatidylcholine, and a coupler of the trinder reagent to a specimen. Such an aspect is also simply referred to as "first aspect of measurement" hereinafter.

Preferred aspects of the ferrocyanide, the trinder reagent, the choline oxidase, the peroxidase, the lysophosphatidylcholine, and the coupler of the trinder reagent, and preferred concentration of each of the components after mixing with the specimen are as described above.

According to the first aspect of the measurement, the following blue coloring agent is produced by the following reaction. Here, the amount of the blue coloring agent is measured by a colorimetric method (for example, an absorbance measurement or the like), thereby the amount of choline produced can be measured. By dividing the amount of choline produced by the reaction time, the amount of choline produced per unit time can be obtained, and the activity of the lysophospholipase D in the specimen can be measured.

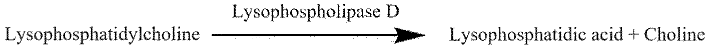

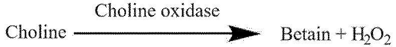

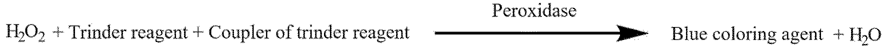

In the first aspect of the measurement, the activity measurement of the lysophospholipase D can be performed, for example, by adding Composition 1 and Composition 2 to a specimen.

Composition 1: a trinder reagent, a choline oxidase, and a peroxidase are contained.

Composition 2: a ferrocyanide, lysophosphatidylcholine, and a coupler of a trinder reagent are contained.

Here, Composition 1 and Composition 2 may be added to the specimen at the same time or within a short time, but the color development (coloration) reaction can be performed after the choline derived from the specimen is removed, by adding Composition 1 first, and adding Composition 2 after a certain period of time. Here, to remove the hydrogen peroxide generated by the reaction between the choline derived from the specimen and the choline oxidase in Composition 1, for example, Composition 1 may contain a component for decomposing hydrogen peroxide, such as catalase, or a component for removing hydrogen peroxide may be added after the addition of the composition 1 and before the addition of Composition 2. In addition, the hydrogen peroxide generated by the reagent 1 and the hydrogen peroxide finally generated by the action of the lysophospholipase D after the addition of the reagent 2 have different generation timings. Therefore, for example, the activity of the lysophospholipase D can be evaluated by taking into consideration the time until hydrogen peroxide is generated by the action of lysophospholipase D, even in a case where the hydrogen peroxide generated by the reagent 1 is not removed, by subtracting the influence of the above-described hydrogen peroxide. Examples the activity measurement include a method of adding the composition 1 to react the mixture for 30 seconds to 30 minutes, and then adding Composition 2.

Details of Composition 1 and Composition 2 will be described below.

In addition, in the first aspect of the measurement, the activity measurement of the lysophospholipase D can be performed, for example, by adding a sensitivity improver for lysophospholipase D activity measurement containing a ferrocyanide, Composition 3, and Composition 4 to the specimen.

Composition 3: a trinder reagent, a choline oxidase, and a peroxidase are contained.

Composition 4: lysophosphatidylcholine, and a coupler of a trinder reagent are contained.

Here, the above-described sensitivity improver, Composition 3, and Composition 4 may be added to the specimen at the same time or within a short time, but color development reaction can be performed after the choline derived from the specimen is removed, by adding Composition 3 first, and adding Composition 4 after a certain period of time. In this case, the sensitivity improver can be added, for example, before the addition of Composition 4 containing the lysophosphatidylcholine as the substrate, or at the same time as the addition of Composition 4. Examples the activity measurement include a method of adding the composition 3 to react the mixture for 30 seconds to 30 minutes, and then adding Composition 4. In the above-described aspect, the sensitivity improver can be added before the addition of Composition 4 or at the same time as the addition of Composition 4. Here, to remove the hydrogen peroxide generated by the reaction between the choline derived from the specimen and the choline oxidase in Composition 3, for example, Composition 3 may contain a component for decomposing hydrogen peroxide, such as catalase, or a component for decomposing hydrogen peroxide may be added after the addition of the composition 3 and before the addition of Composition 4. In addition, the hydrogen peroxide generated by the reagent 3 and the hydrogen peroxide finally generated by the action of the lysophospholipase D after the addition of the reagent 4 have different generation timings. Therefore, for example, the activity of the lysophospholipase D can be evaluated by taking into consideration the time until hydrogen peroxide is generated by the action of lysophospholipase D, even in a case where the hydrogen peroxide generated by the reagent 3 is not removed, by subtracting the influence of the above-described hydrogen peroxide.

Details of the sensitivity improver, Composition 3 and Composition 4 will be described below.

In addition, in the first aspect of the measurement, the activity measurement of the lysophospholipase D can be also performed, for example, by adding Composition 5 to a specimen.

Composition 5: a trinder reagent, a choline oxidase, a peroxidase, a ferrocyanide, lysophosphatidylcholine, and a coupler of the trinder reagent are contained.

In such an aspect, two color development reactions proceed that are a color development reaction (color development reaction 1) proceeding with the choline derived from the specimen and a color development reaction (color development reaction 2) proceeding with the choline produced from the lysophospholipase D and lysophosphatidylcholine in the specimen. However, in a case where Composition 5 is added to the specimen, the color development reaction 1 proceeds rapidly, whereas the color development reaction 2 proceeds at a rate of the lysophospholipase D activity, and proceeds slowly. In this way, since there is a time difference in the color development by each color development reaction, by taking into consideration the time difference, the activity of lysophospholipase D can be also evaluated by subtracting the influence of the color development by choline derived from the specimen (that is, the influence of the color development reaction 1).

Details of Composition 5 will be described later.

In addition, in the first aspect of the measurement, the pretreatment of the specimen may be performed by another method, and the choline derived from the specimen may be removed in advance.

### [Second aspect of measurement of lysophospholipase D activity]

The measurement of the lysophospholipase D activity according to the embodiment of the present invention is preferably performed by adding a ferrocyanide, lysophosphatidylglycerol, a glycerol kinase, a first nucleotide coenzyme, and a second nucleotide coenzyme, the glycerol kinase catalyzing a forward reaction of generating a phosphorylated product of glycerol from the glycerol and a reverse reaction thereof in the presence of a nucleotide coenzyme, using one of the first nucleotide coenzyme and the second nucleotide coenzyme in the forward reaction, and using one of the first nucleotide coenzyme and the second nucleotide coenzyme, which is not used in the forward reaction, in the reverse reaction, the second nucleotide coenzyme having different nucleoside moiety from the nucleoside moiety of the first nucleotide coenzyme. Such an aspect will be also simply referred to as "second aspect of measurement" hereinafter.

According to a second aspect of the measurement, the phosphorylated product of the second nucleotide coenzyme is produced by the following reaction. The amount of glycerol produced can be measured by measuring the amount of the phosphorylated product of the second nucleotide coenzyme. By dividing the amount of glycerol produced by the reaction time, the amount of glycerol produced per unit time can be obtained, and the activity of the lysophospholipase D in the specimen can be measured.

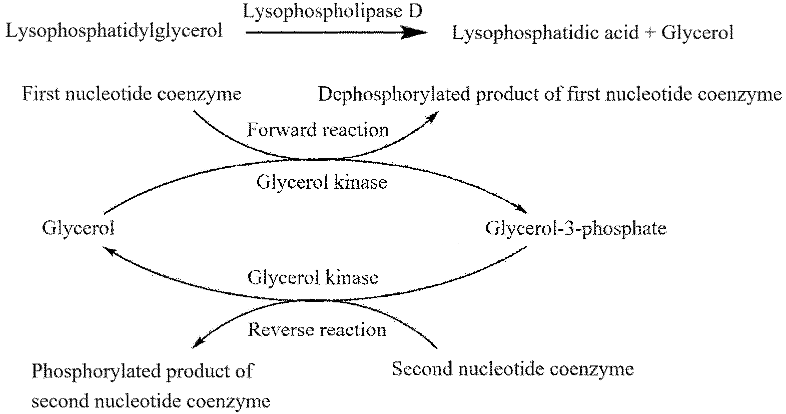

In the second aspect of the measurement, the amount of glycerol produced can be measured by detecting the change amount in one or two or more of the first nucleotide coenzyme, the dephosphorylated product of the first nucleotide coenzyme, the above-described second nucleotide coenzyme, and the phosphorylated product of the second nucleotide coenzyme.

The details of the second aspect of the measurement can be performed according to JP2017-038569A.

### (Sensitivity improver for lysophospholipase D activity measurement)

The sensitivity improver for lysophospholipase D activity measurement according to the embodiment of the present invention (also simply referred to as a "sensitivity improver" in the present specification) contains a ferrocyanide.

The sensitivity improver is preferably a sensitivity improver for the above-described activity measurement of the lysophospholipase D according to the embodiment of the present invention.

The sensitivity improver may be ferrocyanide alone or a composition containing ferrocyanide.

Preferred aspects of the ferrocyanide are the same as the preferred aspects of the ferrocyanide in the above-described activity measurement method of lysophospholipase D according to the present invention.

The amount of the ferrocyanide in a case where the sensitivity improver is a ferrocyanide alone, and the concentration of the ferrocyanide in a case where the sensitivity improver is a composition may be determined according to the concentration used in the measurement of lysophospholipase D activity. As the concentration used in the measurement of lysophospholipase D activity, the concentration described in the measurement of lysophospholipase D activity according to the present invention can be referred.

In a case where the sensitivity improver is a composition, the sensitivity improver may further contain a solvent, a pH buffer agent, and other additives as components different from the ferrocyanide.

Examples of the solvent include water, a water-soluble organic solvent, and a mixture thereof, and water is preferable.

Examples of the pH buffer agent include a phosphate buffer agent, a citrate buffer agent, an acetate buffer agent, a tris-hydrochloride buffer agent, a good buffer agent such as PIPES, MES, and HEPES, and another compound used as a chemical buffer agent.

Examples of other additives include a preservative, an antioxidant, a stabilizer, and the like. As these compounds, known compounds can be used without any particular limitation within a range in which the effects of the present invention are exhibited.

### (Composition)

The composition according to the embodiment of the present invention is a composition for activity measurement of lysophospholipase D, which contains a ferrocyanide and a substrate of lysophospholipase D.

The composition according to the embodiment of the present invention is preferably a composition for the above-described activity measurement of lysophospholipase D according to the present invention.

Preferred aspects of the ferrocyanide and the substrate of the lysophospholipase D contained in the composition according to the embodiment of the present invention are the same as the preferred aspects of the ferrocyanide and the substrate of the lysophospholipase D in the above-described activity measurement method for lysophospholipase D according to the embodiment of the present invention.

For example, the above-described substrate of the lysophospholipase D is preferably lysophosphatidylcholine.

The composition according to the embodiment of the present invention may contain at least one selected from the group consisting of the above-described coupler of the trinder reagent, the above-described trinder reagent, the above-described choline oxidase, and the above-described peroxidase.

Preferred aspects of these compounds are the same as the preferred aspects of these compounds in the above-described activity measurement method for lysophospholipase D according to the embodiment of the present invention.

In addition, the composition according to the embodiment of the present invention may further contain a solvent, a pH buffering agent, and other additives.

Examples of the solvent include water, a water-soluble organic solvent, and a mixture thereof, and water is preferable.

Examples of the pH buffer agent include a phosphate buffer agent, a citrate buffer agent, an acetate buffer agent, a tris-hydrochloride buffer agent, a good buffer agent such as PIPES, MES, and HEPES, and another compound used as a chemical buffer agent.

Examples of other additives include a preservative, an antioxidant, a stabilizer, and the like. As these compounds, known compounds can be used without any particular limitation within a range in which the effects of the present invention are exhibited.

In addition, the concentration of each component contained in the composition may be determined according to the concentration of each component used in the measurement of the lysophospholipase D activity. As the concentration of each component used in the measurement of lysophospholipase D activity, the concentration of each component described in the above-described measurement of lysophospholipase D activity according to the present invention can be referred.

### (Kit)

The kit according to the embodiment of the present invention includes two or more reagents in which the above-described composition according to the embodiment of the present invention or the component contained in the composition according to the embodiment of the present invention are distributed.

The kit according to the embodiment of the present invention is a kit for activity measurement of lysophospholipase D, and is preferably a kit for activity measurement of the lysophospholipase D according to the present invention.

The kit according to the embodiment of the present invention is preferably a reagent kit in which the composition according to the embodiment of the present invention is appropriately distributed into two or more reagents (a component alone or a composition). Here, the kit according to the embodiment of the present invention preferably includes two or more reagents, and all the components contained in the composition according to the embodiment of the present invention are preferably included in any of the above two or more reagents. Each of the components contained in the composition according to the embodiment of the present invention may be included in a plurality of reagents separated from each other.

In addition, the kit according to the embodiment of the present invention may include the composition according to the embodiment of the present invention. For example, the kit according to the embodiment of the present invention is also preferably a kit including a composition which contains a ferrocyanide, lysophosphatidylcholine, a coupler of a trinder reagent, a trinder reagent, a choline oxidase, and a peroxidase. Here, such a kit may be a kit including only one reagent which is the above-described composition.

Here, it is also preferable that the kit according to the embodiment of the present invention includes a reagent that contains at least the choline oxidase and does not substantially contain at least one of the compounds required for the color development, and a reagent that contains the compound required for the color development, which is not substantially contained in the reagent.

For example, the kit according to the embodiment of the present invention also preferably includes a reagent (reagent 1) that contains at least the choline oxidase and does not substantially contain the coupler of the trinder reagent, and a reagent (reagent 2) that contains the coupler of the trinder reagent.

The ferrocyanide, the peroxidase, and the trinder reagent may be contained in any of the reagent 1 or the reagent 2, may be contained in both the reagent 1 and the reagent 2, or may be further contained in another reagent. In a case of being further contained in another reagent, the ferrocyanide, the peroxidase, and the trinder reagent may be distributed to a plurality of reagents or may be contained in the same reagent.

However, for purpose of preventing the color development reaction from proceeding during storage of the reagent, it is possible to avoid an aspect in which the trinder reagent and the coupler of the trinder reagent are contained in one reagent. In particular, an aspect in which the trinder reagent, the coupler of the trinder reagent, and the peroxidase are all contained can also be avoided.

In the present invention, the term "substantially not contained" means that the content is 1 µM or less, and the content is preferably 0.1 µM or less and more preferably 0.01 µM or less. The lower limit of the content is not limited, and may be 0 µM or more.

According to such an aspect, first, the reagent 1 is added to the specimen to decompose and remove choline in the specimen by choline oxidase under the condition that the coupler of the trinder reagent is not present and the color development reaction does not proceed, and then the color development reaction can proceed by adding the reagent 2.

In addition, to remove hydrogen peroxide generated by choline and choline oxidase in the above-described specimen, the addition of the reagent 2 may be spaced from the addition of the reagent 1 in time, or the reagent 1 may further contain a compound for removing hydrogen peroxide, such as catalase.

Specific examples of the kit according to the embodiment of the present invention are shown below, but the kit according to the embodiment of the present invention is not limited thereto. For example, the composition may be further divided into a plurality of reagents, for example, such that Composition 1, which will be described later, is divided into two reagents of a reagent further containing a trinder reagent and a reagent containing choline oxidase and peroxidase. In addition, composition may be further divided into a plurality of reagents, for example, such that Composition 2, which will be described later, is divided into two reagents of a reagent containing a ferrocyanide and a reagent containing lysophosphatidylcholine and a coupler of a trinder reagent.

### [First aspect of kit]

The kit according to the embodiment of the present invention preferably includes Composition 1 and Composition 2.

Composition 1: a trinder reagent, a choline oxidase, and a peroxidase are contained.

Composition 2: a ferrocyanide, lysophosphatidylcholine, and a coupler of a trinder reagent are contained.

A preferred aspect of the activity measurement method of the lysophospholipase D using Composition 1 and Composition 2 is as described in the first aspect of the measurement described above.

### -Composition 1-

Preferred aspects of the trinder reagent, the choline oxidase, and the peroxidase in Composition 1 are the same as the preferred aspects of these compounds in the above-described activity measurement method for lysophospholipase D according to the embodiment of the present invention.

Composition 1 may further contain a solvent, a pH buffering agent, and other additives. Preferred aspects of these compounds are the same as the preferred aspects of these compounds in the above-described composition according to the embodiment of the present invention.

In addition, the concentration of each component contained in Composition 1 may be determined according to the concentration of each component used in the measurement of the lysophospholipase D activity. As the concentration of each component used in the measurement of lysophospholipase D activity, the concentration of each component described in the above-described measurement of lysophospholipase D activity according to the present invention can be referred.

### -Composition 2-

Preferred aspects of the ferrocyanide, lysophosphatidylcholine, and coupler of trinder reagent in Composition 2 are the same as the preferred aspects of these compounds in the above-described activity measurement method for lysophospholipase D according to the embodiment of the present invention.

Composition 2 may further contain a solvent, a pH buffering agent, and other additives. Preferred aspects of these compounds are the same as the preferred aspects of these compounds in the above-described composition according to the embodiment of the present invention.

In addition, the concentration of each component contained in Composition 2 may be determined according to the concentration of each component used in the measurement of the lysophospholipase D activity. As the concentration of each component used in the measurement of lysophospholipase D activity, the concentration of each component described in the above-described measurement of lysophospholipase D activity according to the present invention can be referred.

### [Second aspect of kit]

The kit according to the embodiment of the present invention preferably includes the sensitivity improver according to the embodiment of the present invention, Composition 3, and Composition 4.

Composition 3: a trinder reagent, a choline oxidase, and a peroxidase are contained.

Composition 4: lysophosphatidylcholine, and a coupler of a trinder reagent are contained.

A preferred aspect of the activity measurement method of the lysophospholipase D using Composition 3 and Composition 4 is as described in the first aspect of the measurement described above.

### -Composition 3-

Preferred aspects of the trinder reagent, the choline oxidase, and the peroxidase in Composition 3 are the same as the preferred aspects of these compounds in the above-described activity measurement method for lysophospholipase D according to the embodiment of the present invention.

Composition 3 may further contain a solvent, a pH buffering agent, and other additives. Preferred aspects of these compounds are the same as the preferred aspects of these compounds in the above-described composition according to the embodiment of the present invention.

In addition, the concentration of each component contained in Composition 3 may be determined according to the concentration of each component used in the measurement of the lysophospholipase D activity. As the concentration of each component used in the measurement of lysophospholipase D activity, the concentration of each component described in the above-described measurement of lysophospholipase D activity according to the present invention can be referred.

### -Composition 4-

Preferred aspects of the lysophosphatidylcholine and the trinder reagent in Composition 4 are the same as the preferred aspects of these compounds in the above-described activity measurement method for lysophospholipase D according to the embodiment of the present invention.

Composition 4 may further contain a solvent, a pH buffering agent, and other additives. Preferred aspects of these compounds are the same as the preferred aspects of these compounds in the above-described composition according to the embodiment of the present invention.

In addition, the concentration of each component contained in Composition 4 may be determined according to the concentration of each component used in the measurement of the lysophospholipase D activity. As the concentration of each component used in the measurement of lysophospholipase D activity, the concentration of each component described in the above-described measurement of lysophospholipase D activity according to the present invention can be referred.

### [Third aspect of kit]

The kit according to the embodiment of the present invention also preferably includes Composition 5.

Composition 5: a trinder reagent, a choline oxidase, a peroxidase, a ferrocyanide, lysophosphatidylcholine, and a coupler of the trinder reagent are contained.

A preferred aspect of the activity measurement method of the lysophospholipase D using Composition 5 is as described in the first aspect of the measurement described above.

### -Composition 5-

Preferred aspects of the trinder reagent, the choline oxidase, the peroxidase, the ferrocyanide, the lysophosphatidylcholine, and the coupler of the trinder reagent in Composition 5 are the same as the preferred aspects of these compounds in the above-described activity measurement method for lysophospholipase D according to the embodiment of the present invention.

Composition 5 may further contain a solvent, a pH buffering agent, and other additives. Preferred aspects of these compounds are the same as the preferred aspects of these compounds in the above-described composition according to the embodiment of the present invention.

In addition, the concentration of each component contained in Composition 5 may be determined according to the concentration of each component used in the measurement of the lysophospholipase D activity. As the concentration of each component used in the measurement of lysophospholipase D activity, the concentration of each component described in the above-described measurement of lysophospholipase D activity according to the present invention can be referred.

In addition, the composition according to the embodiment of the present invention or the kit according to the embodiment of the present invention may be incorporated into a specimen measurement device.

Examples of such a device include a device including a system for measuring the lysophospholipase D activity by using, as a specimen, a body fluid (for example, blood, urine, cerebrospinal fluid, pleural effusion, ascites, puncture fluid or an extracted substance thereof, or the like), a tissue (for example, a biopsy tissue or an extracted substance thereof, or the like), a lysophospholipase D solution, a cultured cell or an extracted substance thereof, a cell culture supernatant, or the like, with the composition according to the embodiment of the present invention or the kit according to the embodiment of the present invention.

For example, the device may be a device in which the lysophospholipase D activity is measured using the body fluid as a clinical specimen, such as a self-blood sugar measuring device. That is, the device may be a device used for point-of-care testing (POCT), that is, clinical tests in medical fields such as medical care and nursing.

In addition, the device may be a device including a specimen collection system and a specimen treatment system.

### Examples

Hereinafter, the present invention will be described in detail using examples. Materials, using amounts, proportions, treatment details, treatment content, and the like shown in the following examples can be appropriately changed without departing from the gist of the present invention. Therefore, the scope of the present invention is not limited to the specific examples described below.

### (Comparative Example 1 and Examples 1 to 8: verification of sensitivity improvement effect by potassium ferrocyanide in activity measurement of lysophospholipase D of ATX)

The sensitivity improvement effect of potassium ferrocyanide in the measurement of the lysophospholipase D activity of ATX was verified. The measurement reagent was based on 100 mM Tris-HCl (pH 9.0), 5 mM magnesium chloride hexahydrate (FUJIFILM Wako Pure Chemical Corporation), 0.05% (w/v) Triton X-100 (FUJIFILM Wako Pure Chemical Corporation), 1 mM myristoyl lysophosphatidylcholine (Asahi Kasei Pharma), 2 mM 4-aminoantipyrine (FUJIFILM Wako Chemical Corporation), 1 mM N-Ethyl-N-(3-sulfopropyl)-3-methylaniline (TOPS, sodium salt, monohydrate, Dojindo Laboratories), 2 U/mL choline oxidase (Asahi Kasei Pharma), and 10 U/mL peroxidase (TOYOBO), and 70 µL of the measurement reagent containing potassium ferrocyanide (FUJIFILM Wako Pure Chemical Corporation) at a concentration shown in Table 1 was used in each of Examples and Comparative Examples. 7 µL of a pool serum to which a recombinant ATX (Asahi Kasei Pharma, human autotaxin β isoform) was added was used as a measurement target, and using an automatic analyzer JCA-BM9130 (JEOLLtd.) and the above-described measurement reagent, an absorbance change (ΔE/min) for 2 to 7 minutes after the start of the measurement at a photometric wavelength of 545 nm and under the analysis condition of an initial reaction rate assay (RRA). In the enzyme activity of ATX, an activity to produce 1 µmol of choline from myristoyl lysophosphatidylcholine as a substrate at 37°C for 1 minute was defined as 1 unit (U) based on J. Cell Biol., 158, 227-233 (2002). The measurement results are shown in Table 1. In the table, the sensitivity is a value obtained by subtracting the measurement sensitivity of physiological saline from the measurement sensitivity of the sample, and "vs 0 mM" is a relative value in which the sensitivity in a case of not containing potassium ferrocyanide (Comparative Example 1) is set to 100%.

**[Table 1]**

| | | Comparative Example 1 | Example 1 | Example 2 | Example 3 | Example 4 | Example 5 | Example 6 | Example 7 | Example 8 |
|---|---|---|---|---|---|---|---|---|---|---|
| Potassium ferrocyanide (mM) | | 0.000 | 0.005 | 0.010 | 0.050 | 0.100 | 0.500 | 1.000 | 5.000 | 10.000 |
| ΔE/min*10000 | | | | | | | | | | |
| Per ATX 20 U/L | Sensitivity | 22 | 36 | 36 | 41 | 43 | 47 | 49 | 50 | 52 |
| | vs 0 mM | vs | 164% | 164% | 186% | 195% | 214% | 223% | 227% | 236% |

From the results shown in Table 1, it was found that, in the lysophospholipase D activity measurement of ATX, the sensitivity was significantly improved by 1.5 times or more by coexisting potassium ferrocyanide (Comparative Example 1, Examples 1 to 8).

(Comparative Example 2 and Examples 9 to 16: verification of improvement effect of simultaneous reproducibility by potassium ferrocyanide in activity measurement of lysophospholipase D of ATX)

The same specimen was measured 5 times by the same method as in Comparative Example 1 and Examples 1 to 8, and the improvement effect of the simultaneous reproducibility by potassium ferrocyanide was evaluated by a coefficient of variation CV (%). The results are shown in Table 2.

**[Table 2]**

| | | Comparative Example 2 | Example 9 | Example 10 | Example 11 | Example 12 | Example 13 | Example 14 | Example 15 | Example 16 |
|---|---|---|---|---|---|---|---|---|---|---|
| Potassium ferrocyanide (mM) | | 0.000 | 0.005 | 0.010 | 0.050 | 0.100 | 0.500 | 1.000 | 5.000 | 10.000 |
| U/L | | | | | | | | | | |
| Measurement times | 1 | 9.2 | 9.9 | 9.2 | 9.2 | 9.2 | 9.2 | 9.2 | 9.2 | 9.5 |
| | 2 | 9.2 | 8.5 | 9.2 | 8.7 | 9.2 | 9.2 | 9.2 | 9.2 | 9.2 |
| | 3 | 9.2 | 9.2 | 8.6 | 9.2 | 9.7 | 8.8 | 9.2 | 9.2 | 9.2 |
| | 4 | 9.2 | 9.9 | 9.2 | 7.6 | 9.2 | 9.2 | 9.6 | 9.2 | 9.2 |
| | 5 | 6.1 | 9.9 | 8.0 | 8.7 | 8.7 | 9.2 | 9.6 | 9.6 | 8.9 |
| Average [U/L] | | 8.6 | 9.5 | 8.8 | 8.7 | 9.2 | 9.1 | 9.4 | 9.3 | 9.2 |
| Standard deviation | | 1.39 | 0.63 | 0.54 | 0.65 | 0.35 | 0.18 | 0.22 | 0.18 | 0.21 |
| Coefficient of variation CV [%] | | 16.2% | 6.6% | 6.1% | 7.5% | 3.8% | 2.0% | 2.3% | 1.9% | 2.3% |

The simultaneous reproducibility is an indicator of how much the measurement results vary in a case where the same specimen is repeatedly measured within a short time, and is one of the basic performances of the test reagent. It can be said that, as the reagent for a general-purpose automatic analysis apparatus, a coefficient of variation CV(%) in a case where the same specimen is usually measured 5 times is preferably 10% or less. In addition, it can be said to be preferable that the smaller the above-described CV is, the better the simultaneous reproducibility is. From Table 2, the coefficient of variation in a case where the enzyme activity of ATX was measured without using potassium ferrocyanide was 16.2%, and the simultaneous reproducibility was low (Comparative Example 2). On the other hand, in a case where the enzyme activity of ATX was measured in the presence of potassium ferrocyanide, the CV was 10% or less in a case of potassium ferrocyanide of 0.005 mM or more, and the improvement of the simultaneous reproducibility was observed. It was found that CV was 5% or less in a case of potassium ferrocyanide of 0.1 mM or more, thereby the potassium ferrocyanide was particularly useful.

### (Comparative Example 3 and Examples 17 and 18: verification of sensitivity improvement effect by sodium ferrocyanide in activity measurement of lysophospholipase D of ATX)

The sensitivity improvement effect of sodium ferrocyanide in the measurement of the lysophospholipase D activity of ATX was verified. In Comparative Example 3 and Examples 17 and 18, the measurement was performed in the same manner as in Example 1, except that 70 µL of each solution containing sodium ferrocyanide (manufactured by FUJIFILM Wako Pure Chemical Corporation) to have a concentration shown in Table 3 was used instead of potassium ferrocyanide.

The measurement results are shown in Table 3. In the table, the sensitivity is a value obtained by subtracting the measurement sensitivity of physiological saline from the measurement sensitivity of the sample, and "vs 0 mM" is a relative value in which the sensitivity in a case of not containing sodium ferrocyanide (Comparative Example 3) is set to 100%.

**[Table 3]**

| | Comparative Example 3 | Example 17 | Example 18 |
|---|---|---|---|
| Sodium ferrocyanide (mM) | 0.00 | 0.01 | 1.00 |
| ΔE/min × 10000 | | | |
| Sensitivity per ATX 20 U/L | 26 | 39 | 45 |
| vs 0 mM | vs | 150.0% | 173.1% |

From the results shown in Table 3, it was found that, in the measurement of the lysophospholipase D activity of ATX, the sensitivity was significantly improved by 1.5 times or more by coexisting sodium ferrocyanide (Comparative Example 3, Examples 17 and 18).

### (Comparative Example 4 and Examples 19 to 21: verification of sensitivity improvement effect in case where each component of composition is distributed to two reagents)

The measurement reagent was prepared at a concentration shown in Table 4. The amount of the reagent used for the measurement was 100 µL in Comparative Example 4 and Example 19, and a total of 100 µL of 50 µL of the reagent 3 and 50 µL of the reagent 4 in Example 20. In Example 20, to match the final concentration of each component with that of Example 19, the concentration of the choline oxidase, the TOPS, the peroxidase, the 4-aminoantipyrine, and the myristoyl lysophosphatidylcholine, which are reagents contained in only one of the reagent 3 and the reagent 4, was doubled the concentration in the reagent 2 of Example 19. 10 µL of a pool serum to which a recombinant ATX (Asahi Kasei Pharma, human autotaxin β isoform) was added was used as a measurement target, and using an automatic analyzer JCA-BM9130 (manufactured by JEOL Ltd.) and the above-described measurement reagent, an absorbance change (ΔE/min) for 6 to 7 minutes after the start of the measurement at a photometric wavelength of 545 nm and under the analysis condition of an initial reaction rate assay (RRA). In the enzyme activity of ATX, an activity to produce 1 µmol of choline from myristoyl lysophosphatidylcholine as a substrate at 37°C for 1 minute was defined as 1 unit (U) based on J. Cell Biol., 158, 227-233 (2002). The measurement results are shown in Table 5. In the table, the sensitivity is a value obtained by subtracting the measurement sensitivity of physiological saline from the measurement sensitivity of the sample, and "vs 0 mM" is a relative value in which the sensitivity in a case of not containing potassium ferrocyanide (Comparative Example 4) is set to 100%.

**[Table 4]**

| | Comparative Example 4 | Example 19 | Example 20 | |
|---|---|---|---|---|
| | Reagent 1 | Reagent 2 | Reagent 3 | Reagent 4 |
| Component | Concentration | Concentration | Concentration | Concentration |
| Tris-HCl(pH9.0) | 100 mM | 100 mM | 100 mM | 100 mM |
| Magnesium chloride hexahydrate | 5 mM | 5 mM | 5 mM | 5 mM |
| Triton X-100 | 0.05% (w/v) | 0.05% (w/v) | 0.05% (w/v) | 0.05% (w/v) |
| Choline oxidase | 2 U/mL | 2 U/mL | 4 U/mL | - |
| TOPS | 1 mM | 1 mM | 2 mM | - |
| Peroxidase | 10 U/mL | 10 U/mL | 20 U/mL | - |
| 4-Aminoantipyrine | 2 mM | 2 mM | - | 4 mM |
| Myristoyl lysophosphatidylcholine | 1 mM | 1 mM | - | 2 mM |
| Potassium ferrocyanide | - | 1 mM | - | 2 mM |

**[Table 5]**

| | Comparative Example 4 | Example 19 | Example 20 |
|---|---|---|---|
| ΔE/min × 10000 | | | |
| Sensitivity per ATX 20 U/L | 20 | 34 | 36 |
| vs 0 mM | vs | 170% | 180% |

From the results in Table 5, in the measurement of the lysophospholipase D activity of ATX, in a case where each component of the composition was distributed to two reagents (Example 20), similarly as in a case where each component was contained in one reagent (Example 19), the sensitivity was improved by the ferrocyanide. (Comparative Example 4, Examples 19 and 20).

## Claims

1. An activity measurement method of lysophospholipase D, comprising:
measuring lysophospholipase D activity possessed by the lysophospholipase D, in a presence of a ferrocyanide.

2. The activity measurement method of lysophospholipase D according to claim 1,
wherein the ferrocyanide is potassium ferrocyanide or sodium ferrocyanide.

3. The activity measurement method of lysophospholipase D according to claim 1 or 2,
wherein the measurement includes bringing the lysophospholipase D into contact with a substrate to produce a product.

4. The activity measurement method of lysophospholipase D according to claim 3,
wherein the substrate is lysophosphatidylcholine and the product is choline.

5. The activity measurement method of lysophospholipase D according to claim 1 or 2,
wherein the lysophospholipase D is autotaxin.

6. The activity measurement method of lysophospholipase D according to claim 1 or 2,
wherein a concentration of the ferrocyanide in the measurement of the lysophospholipase D activity is 0.005 mmol/l or more.

7. A sensitivity improver for activity measurement of lysophospholipase D, comprising:
a ferrocyanide.

8. A composition for activity measurement of lysophospholipase D, comprising:
a ferrocyanide; and
a substrate of lysophospholipase D.

9. The composition according to claim 8,
wherein the substrate is lysophosphatidylcholine.

10. The composition according to claim 8 or 9,
wherein a concentration of the ferrocyanide in the activity measurement is 0.005 mmol/l or more.

11. A kit for activity measurement of lysophospholipase D, comprising:
the composition according to claim 8 or 9 or two or more reagents in which components contained in the composition are distributed.
